# EUROPEAN PATENT APPLICATION

(11) **EP 4 606 796 A1**
(43) Date of publication of application: **27.08.2025**
(21) Application number: 23879867.2
(22) Date of filing: 19.10.2023
(51) Int. Cl.: C07D 413/04, A01M 17/00, A01N 47/02, A01P 7/04

(54) **CRYSTAL OF 2-(5-CYCLOPROPYL-3-(ETHYLSULFONYL)PYRIDIN-2-YL)-5-((TRIFLUOROMETHYL)SULFONYL)BENZO[D]OXAZOLE**

(30) Priority: 20.10.2022 JP 2022168599
(71) Applicant: Sumitomo Chemical Company, Limited, Tokyo 103-6020 (JP)
(72) Inventor: MINEGISHI, Hidemitsu, Takarazuka-shi, Hyogo 665-8555 (JP); KAMEZAKI, Masashi, Takarazuka-shi, Hyogo 665-8555 (JP); SHINOMIYA, Hiroto, Takarazuka-shi, Hyogo 665-8555 (JP)
(74) Representative: Vossius & Partner Patentanwälte Rechtsanwälte mbB
(86) International application number: PCT/JP2023/037851
(87) International publication number: WO 2024/085224

(57) **Abstract**

The present provides a Type 1 crystal of 2-(5-cyclopropyl-3-(ethylsulfonyl)pyridin-2-yl)-5-((trifluoromethyl)sulfonyl)benzo[d]oxazole represented by formula (1), the crystal having diffraction peaks at 2θ = 14.3±0.2°, 15.6±0.2°, 17.1±0.2°, 18.7±0.2°, 20.1±0.2°, 22.4±0.2°, 23.3±0.2°, 24.8±0.2°, 26.1±0.2° and 28.5±0.2° in a powder X-ray diffraction with Cu-Kα radiation, which shows an excellent efficacy against harmful organism; a process for preparing the same crystal; a harmful organism control composition comprising the same crystal; as well as a method for controlling harmful organism using the same crystal.

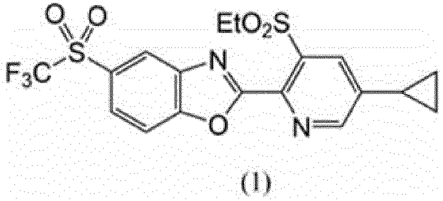

## Description

### TECHNICAL FIELD

This application claims priority to and the benefit of Japanese Patent Application No. 2022-168599 filed October 20,2022, the entire contents of which are incorporated herein by reference.

The present invention relates to a novel crystal of 2-(5-cyclopropyl-3-(ethylsulfonyl)pyridin-2-yl)-5-((trifluoromethyl)sulfonyl)benzo[d]oxazole represented by the following formula (1): 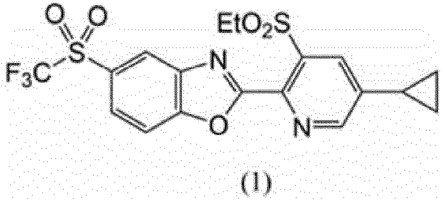 (hereinafter, referred to as "Compound (1)"), showing harmful organism control efficacy. The present invention relates also to a process for preparing the same crystal, a harmful organism control composition comprising the same crystal, and a method for controlling harmful organism using the same crystal.

### BACKGROUND ART

Patent document 1 describes that Compound (1) having a melting point of 177 to 178°C shows a control efficacy against harmful organism.

Also, Patent document 2 and 3 describe a process for preparing Compound (1).

### CITATION LIST

### PATENT DOCUMENT

Patent document 1: WO 2017/146226 A1
Patent document 2: WO 2019/009387 A1
Patent document 3: JP 2020-172469 A1

### SUMMARY OF THE INVENTION

### (PROBLEMS TO BE SOLVED BY INVENTION)

The purpose of the present invention provides a novel crystal of Compound (1) showing excellent control efficacy against harmful organism (hereinafter, referred to as "Type 1 crystal").

### (MEANS TO SOLVE PROBLEMS)

The present inventors have intensively studied to find out a compound showing excellent control efficacy against harmful organism, and as a result, found out that Type 1 crystal of 2-(5-cyclopropyl-3-(ethylsulfonyl)pyridin-2-yl)-5-((trifluoromethyl)sulfonyl)benzo[d]oxazole represented by the following formula (1) as a novel crystal shows an excellent efficacy against harmful organism.

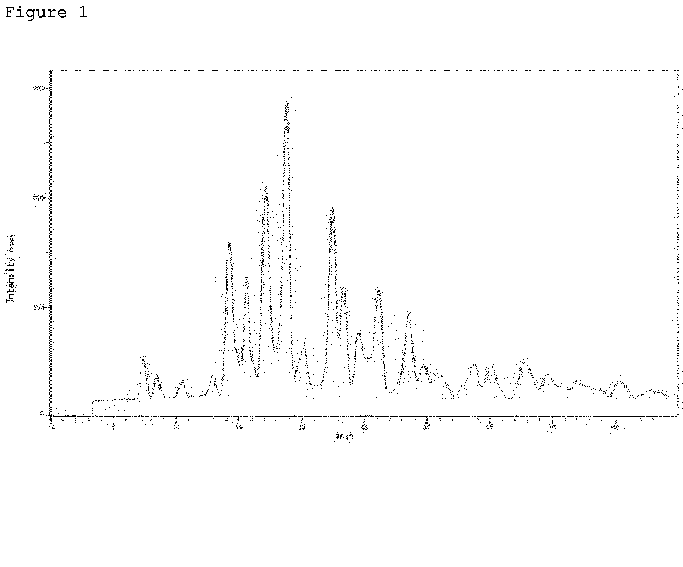

That is, the present invention encompasses the followings.
[1] A Type 1 crystal of 2-(5-cyclopropyl-3-(ethylsulfonyl)pyridin-2-yl)-5-((trifluoromethyl)sulfonyl)benzo[d]oxazole, wherein the crystal has diffraction peaks at 2θ = 14.3±0.2°, 15.6±0.2°, 17.1±0.2°, 18.7±0.2°, 20.1±0.2°, 22.4±0.2°, 23.3±0.2°, 24.8±0.2°, 26.1±0.2°, and 28.5±0.2° in a powder X-ray diffraction with Cu-Kα radiation.
[1-1] A Type 1 crystal of 2-(5-cyclopropyl-3-(ethylsulfonyl)pyridin-2-yl)-5-((trifluoromethyl)sulfonyl)benzo[d]oxazole, wherein the diffraction peaks at 2θ in a powder X-ray diffraction with Cu-Kα radiation has a powder X-ray diffraction pattern described in any one of Figure 1, Figure 3, Figure 5, Figure 7 or Figure 9.
[2] The Type 1 crystal according to [1] wherein a lattice constant measured by single crystal X-ray diffraction is indicated below:
   a = 6.45Å±0.01 Å, b = 14.55 Å±0.01 Å,
   c = 20.86 Å±0.01 Å, α = β =γ = 90°.
[3] The Type 1 crystal according to [1] or [2], wherein the crystal has peaks at positions of 1370±4cm⁻¹, 1317±4cm⁻¹, 1190±4cm⁻¹, 1124±4cm⁻¹, 1093±4cm⁻¹, 1047±4cm⁻¹, 1034±4cm⁻¹, 846±4cm⁻¹, 826±4cm⁻¹, 776±4cm⁻¹, 738±4cm⁻¹ and 719±4cm⁻¹ as a wavenumber in an infrared absorption spectrum (ATR method).
[4] The Type 1 crystal according to any one of [1] to [3], wherein a melting point is 180 to 182 °C.
[5] A method for preparing the Type 1 crystal according to any one of [1] to [4], which comprises
   a step of dissolving 2-(5-cyclopropyl-3-(ethylsulfonyl)pyridin-2-yl)-5-((trifluoromethyl)sulfonyl)benzo[d]oxazole that melts at a range of 175 to 178°C into one or more solvents selected from the group consisting of hydrocarbon, alcohol, ether and ketone, and
   a step of cooling the resulting solution to precipitate a crystal.
[6] A harmful organism control composition comprising the Type 1 crystal according to any one of [1] to [4].
[7] A composition which comprises one or more ingredients selected from the group consisting of the following Groups (a), (b), (c) and (d), as well as the Type 1 crystal according to any one of [1] to [4]:
   Group (a): a group consisting of insecticidal ingredients, miticidal ingredients, and nematicidal ingredients;
   Group (b): fungicidal ingredients;
   Group (c): plant growth modulating ingredients; and
   Group (d): repellent ingredients.
[8] A method for controlling a harmful organism which comprises applying an effective amount of the Type 1 crystal according to any one of [1] to [4] or an effective amount of the harmful organism control composition according to [6] to a harmful organism or a habitat where the harmful organism lives.
[9] A seed or vegetative reproductive organ carrying an effective amount of the Type 1 crystal according to any one of [1] to [4] or an effective amount of the composition according to [6].
[10] Use of the Type 1 crystal according to any one of [1] to [4] for controlling a harmful organism.

### [EFFECT OF INVENTION]

The present invention can provide the Type 1 crystal of Compound (1) showing excellent control efficacy against harmful organism.

### Brief Description of Drawings

[Fig. 1]
   Figure 1 is a figure showing powder X-ray diffraction patterns of the Type 1 crystal of Compound (1) obtained in Example 1. The vertical axis represents diffraction intensity (unit: cps) and the horizontal axis represents diffraction angle (2θ) (unit: °).
[Fig. 2]

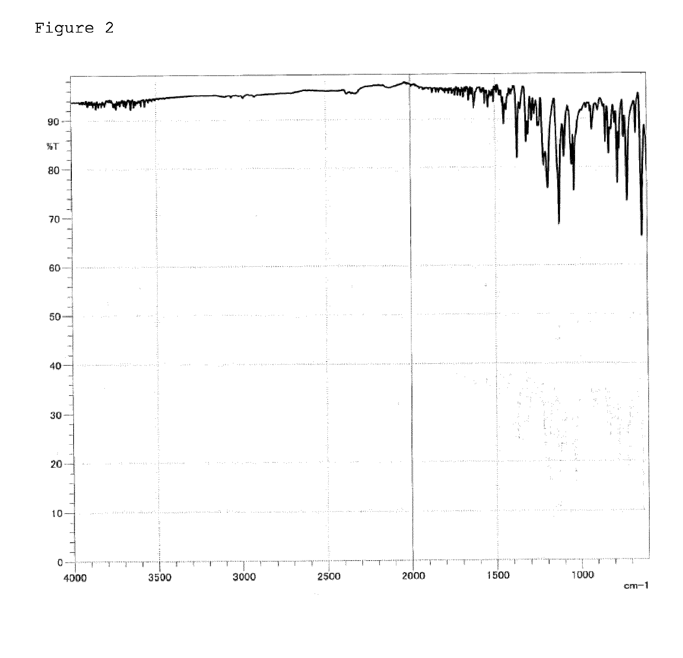

   Figure 2 is a figure showing a spectral diagram of infrared spectrum of the Type 1 crystal of Compound (1) obtained in Example 1. The vertical axis represents a transmittance, and the horizontal axis represents an irradiated infrared wavenumber.
[Fig. 3]

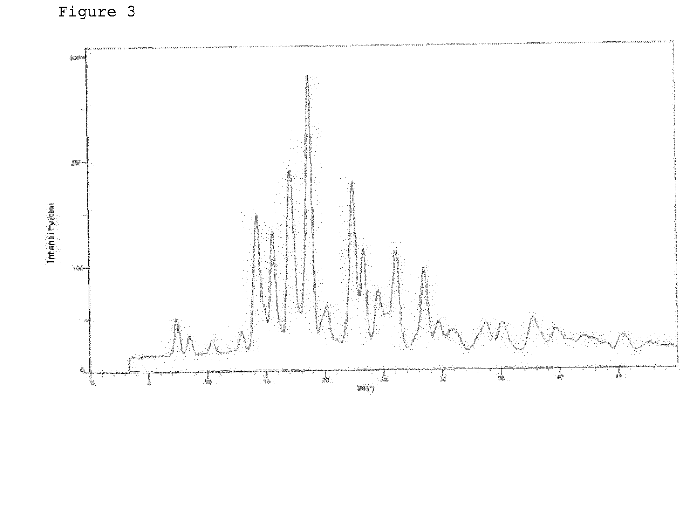

   Figure 3 is a figure showing powder X-ray diffraction patterns of the Type 1 crystal of Compound (1) obtained in Example 2. The vertical axis represents diffraction intensity (unit: cps), and the horizontal axis represents diffraction angle (2θ) (unit: °).
[Fig. 4]

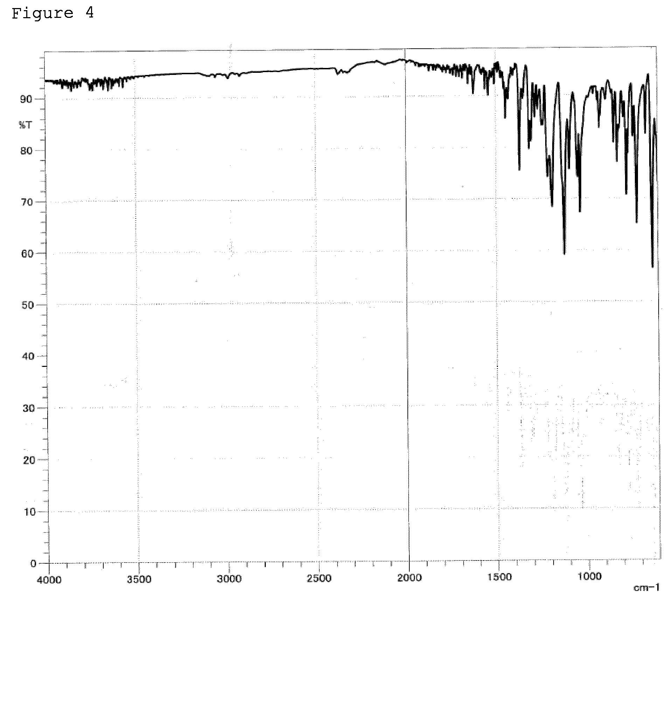

   Figure 4 is a figure showing a spectral diagram of infrared spectrum of the Type 1 crystal of Compound (1) obtained in Example 2. The vertical axis represents a transmittance (unit: %), and the horizontal axis represents an irradiated infrared wavenumber (unit: cm⁻¹).
[Fig. 5]

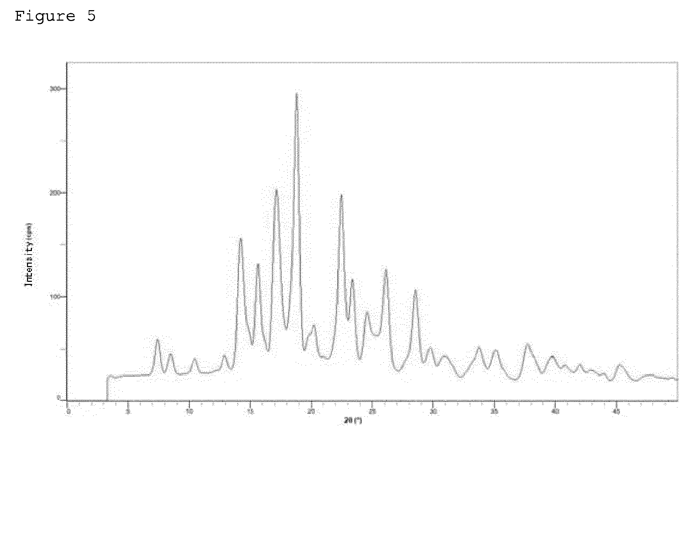

   Figure 5 is a figure showing powder X-ray diffraction patterns of the Type 1 crystal of Compound (1) obtained in Example 3. The vertical axis represents diffraction intensity (unit: cps), and the horizontal axis represents diffraction angle (2θ) (unit: °).
[Fig. 6]

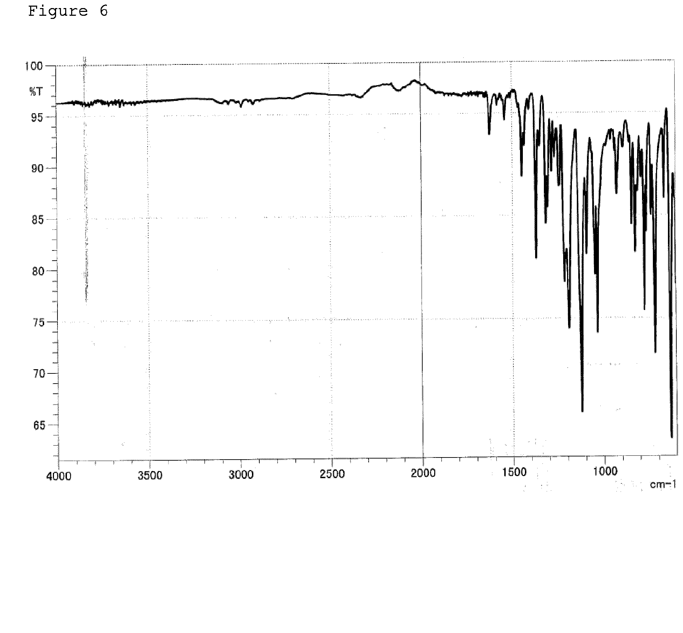

   Figure 6 is a figure showing a spectral diagram of infrared spectrum of the Type 1 crystal of Compound (1) obtained in Example 3. The vertical axis represents a transmittance (unit: %), and the horizontal axis represents an irradiated infrared wavenumber (unit: cm⁻¹).
[Fig. 7]

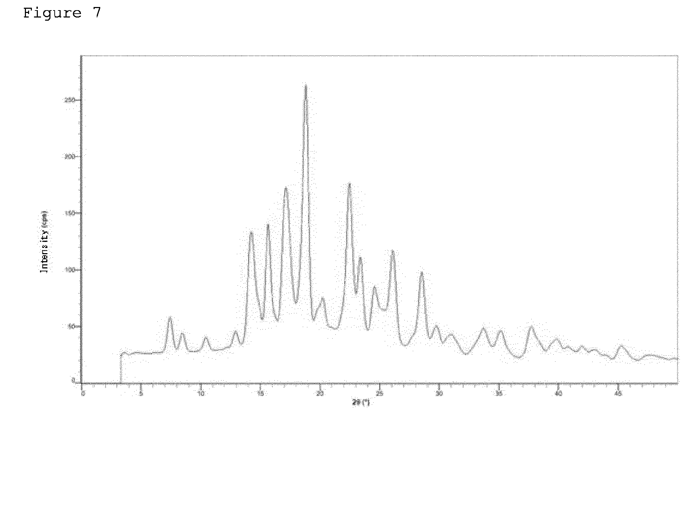

   Figure 7 is a figure showing powder X-ray diffraction patterns of the Type 1 crystal of Compound (1) obtained in Example 4. The vertical axis represents diffraction intensity (unit: cps), and the horizontal axis represents diffraction angle (2θ) (unit: °).
[Fig. 8]

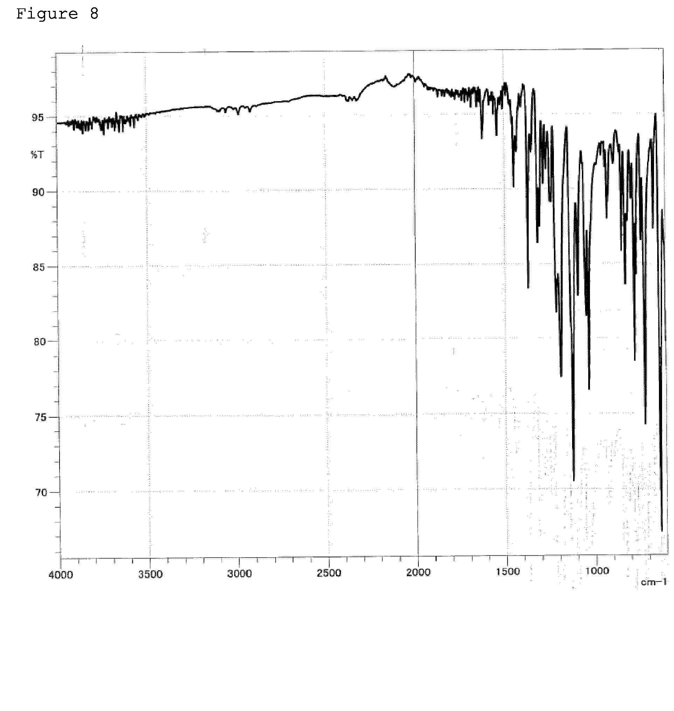

   Figure 8 is a figure showing a spectral diagram of infrared spectrum of the Type 1 crystal of Compound (1) obtained in Example 4. The vertical axis represents a transmittance (unit: %), and the horizontal axis represents an irradiated infrared wavenumber (unit: cm⁻¹).
[Fig. 9]

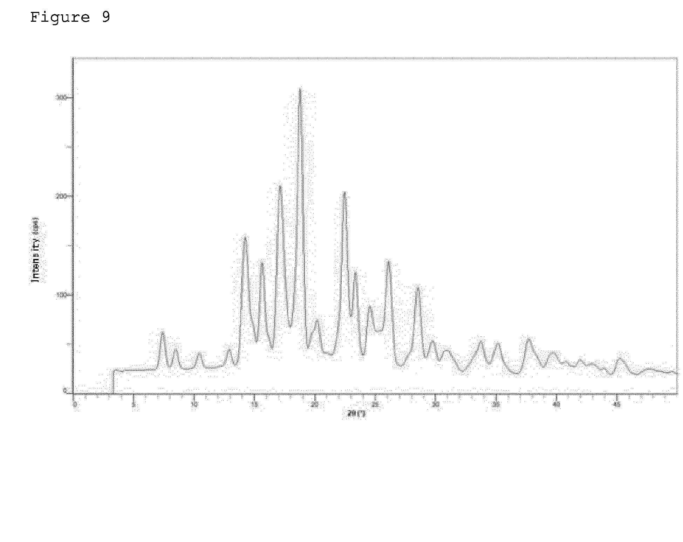

   Figure 9 is a figure showing powder X-ray diffraction patterns of the Type 1 crystal of Compound (1) obtained in Example 5. The vertical axis represents diffraction intensity (unit: cps), and the horizontal axis represents diffraction angle (2θ) (unit: °).
[Fig. 10]

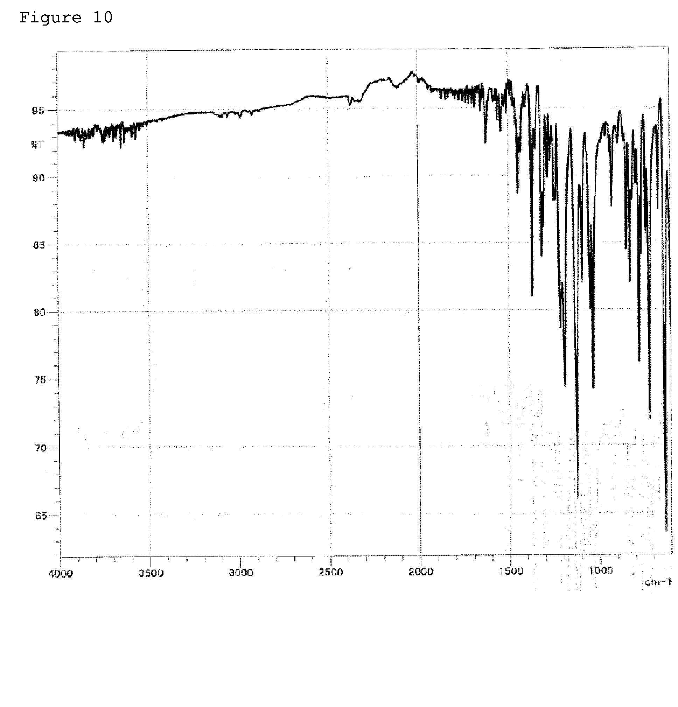

   Figure 10 is a figure showing a spectral diagram of infrared spectrum of the Type 1 crystal of Compound (1) obtained in Example 5. The vertical axis represents a transmittance (unit: %), and the horizontal axis represents an irradiated infrared wavenumber (unit: cm⁻¹).
[Fig. 11]

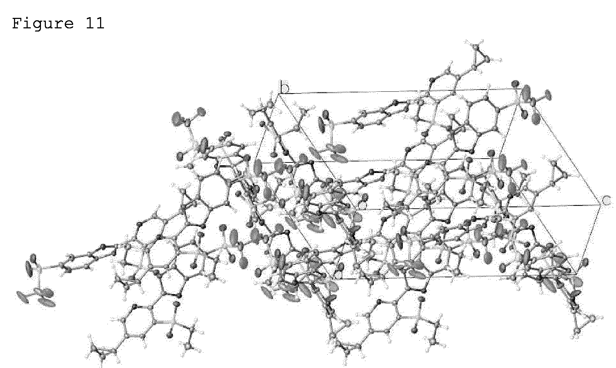

   Figure 11 is a figure showing a molecular packing in a crystal lattice of the Type 1 crystal of Compound (1) obtained in Example 1.

### MODE FOR CARRYING OUT THE INVENTION

The present invention relates to a crystal of Compound (1). The Type 1 crystal of Compound (1) can be prepared according to the following processes.

### 1) Process 1

Compound (1) which is prepared according to the processes described in the Patent document 1, the Patent document 2, or the Patent document 3 is dissolved in solvents at temperatures within a range of 40 to 100°C, and the resulting solutions are cooled gradually (for example, 3°C/hr), and crystals are precipitated at temperatures within a range of 10 to 30°C. If the crystal fails to be precipitated, a part of solvents is evaporated to decrease the amounts of solvent, and then the solutions may be gradually cooled again. The precipitated crystals can be separated to obtain the Type 1 crystal of Compound (1). Examples of the separation methods include a filtration and a centrifugation.

Examples of the solvents used include preferably one or more solvents selected from the group consisting of hydrocarbons, alcohols, ethers, and ketones.

Examples of the hydrocarbons include aromatic hydrocarbons (such as benzene, toluene, xylene, tetrahydronaphthalene and the like); halogenated aromatic hydrocarbons (such as chlorobenzene, o-dichlorobenzene, m-dichlorobenzene, p-dichlorobenzene and the like); nitro aromatic hydrocarbons (such as nitrobenzene and the like); acyclic hydrocarbons (such as hexane, heptane, octane, nonane, decane and the like); aliphatic hydrocarbons including alicyclic hydrocarbons (such as cyclopentane, cyclohexane, methylcyclohexane, cycloheptane and the like); and mixed solvents consisting of two or more of these solvents. Among these solvents, toluene is particularly preferably included.

Examples of alcohols include acyclic hydrocarbon alcohols (such as methanol, ethanol, 1-propanol, 2-propanol, 1-butanol, 2-butanol, 2-methyl-1-propanol, 2-methyl-2-propanol, 3-methyl-1-butanol and the like), and cyclic hydrocarbon alcohols (such as cyclohexanol, benzyl alcohol and the like), and among them, 2-propanol is preferably included.

Examples of ethers include acyclic hydrocarbon ethers (such as diethyl ether, diisopropyl ether, tert-butyl methyl ether, 1,2-dimethoxyethane and the like), and cyclic hydrocarbon ethers (such as tetrahydrofuran, 2-methyl tetrahydrofuran, 1,4-dioxane, methoxy cyclopentane and the like), and among them, tetrahydrofuran and tert-butyl methyl ether are preferably included.

Examples of ketones include acyclic ketones (such as acetone, isoamyl methyl ketone, 2-butanone, 2-pentanone, 3-pentanone, 2-heptanone and the like) and cycic ketones (such as cyclopentanone, cyclohexanone and the like), and among them, acetone is preferably included.

### 2) Process 2

Compound (1) which is prepared according to the processes described in the Patent Literature 1, the Patent Literature 2, or the Patent Literature 3 is dissolved in good solvents at temperatures within a range of 10 to 30°C, and poor solvents are added gradually until solids are precipitated, and crystals are precipitated at temperatures within a range of 10 to 30°C. If the crystal fails to be precipitated, a part of solvents is evaporated to decrease the amounts of solvent, and then the poor solvents may be added gradually again. The precipitated crystals can be separated to obtain the Type 1 crystal of Compound (1). Examples of the separation methods include a filtration and a centrifugation.

Examples of good solvents include one or more solvents selected from the group consisting of aromatic compounds, alcohols, ethers and ketones. Examples of aromatic compounds include aromatic hydrocarbon, halogenated aromatic hydrocarbons, nitro aromatic hydrocarbons and the like. Examples of the aromatic hydrocarbon include aromatic hydrocarbons (such as benzene, toluene, xylene, tetrahydronaphthalene and the like); halogenated aromatic hydrocarbons (such as chlorobenzene, o-dichlorobenzene, m-dichlorobenzene, p-dichlorobenzene and the like); nitro aromatic hydrocarbons (such as nitrobenzene and the like); and mixed solvents comprising two or more of these solvents. Among them, toluene is preferably included. Examples of alcohols include methanol, ethanol, 1-propanol, 2-propanol, 1-butanol, 2-butanol, 2-methyl-1-propanol, 2-methyl-2-propanol, 3-methyl-1-butanol, cyclohexanol, and benzyl alcohol. Examples of the ethers include diethyl ether, diisopropyl ether, tert-butyl methyl ether, tetrahydrofuran, 2-methyl tetrahydrofuran, 1,4-dioxane, 1,2-dimethoxy ethane, and methoxy cyclopentane. Examples of ketones include acyclic ketones (such as acetone, isoamyl methyl ketone, 2-butanone, 2-pentanone, 3-pentanone, 2-heptanone and the like) and cycic ketones (such as cyclopentanone, cyclohexanone and the like).

Examples of the poor solvents include aliphatic hydrocarbons. Examples of the aliphatic hydrocarbons include acyclic hydrocarbons (such as hexane, heptane, octane, nonane, decane and the like), and cyclic hydrocarbons (such as cyclopentane, cyclohexane, methylcyclohexane, cycloheptane and the like). Among them, hexane is preferably included.

The Type 1 crystal of Compound (1) obtained according to the above-mentioned processes has diffraction peaks indicated in Figure 1 as one example of diffraction peaks 2θ (°) in a powder X-ray diffraction with Cu-Kα radiation, and has, for example, diffraction peaks at 2θ (°) = 14.3±0.2°, 15.6±0.2°, 17.1±0.2°, 18.7±0.2°, 20.1±0.2°, 22.4±0.2°, 23.3±0.2°, 24.8±0.2°, 26.1±0.2°, and 28.5±0.2°.

The Type 1 crystal of Compound (1) obtained according to the above-mentioned processes has peaks indicated in Figure 2 as one example of peaks in an infrared absorption spectrum (ATR method), and has, for example, peaks at positions of 1370±4cm⁻¹, 1317±4cm⁻¹, 1190±4cm⁻¹, 1124±4cm⁻¹, 1093±4cm⁻¹, 1047±4cm⁻¹, 1034±4cm⁻¹, 846±4cm⁻¹, 826±4cm⁻¹, 776±4cm⁻¹, 738±4cm⁻¹ and 719±4cm⁻¹ as a wavenumber.

The Type 1 crystal of Compound (1) obtained according to the above-mentioned processes has a melting point of about 180 to 182°C.

The Type 1 crystal of Compound (1) obtained according to the above-mentioned processes has the following crystallographic data of a single crystal when measured at -173°C (100K) by a single crystal X-ray diffraction.
Crystal system: crystal of orthorhombic crystal system Space group: P2₁2₁2₁
Lattice constant: a = 6.45 Å±0.01 Å, b = 14.55 Å±0.01 Å, c = 20.86 Å±0.01 Å, α = β = γ = 90°.
Unit cell volume: V=1958.0±0.1 Å³
Number of molecules in unit cell: Z=4
Density: 1.562 g/cm³

The Type 1 crystal of Compound (1) can be obtained by adding seed crystals of the Type 1 crystal of Compound (1) into supersaturated solutions of Compound (1) having a range of 175 to 178°C as melting point, keeping the temperature or cooling the solutions gradually (for example, 3°C/hr) to precipitate crystals. Examples of the solvents used for preparation of supersaturated solutions may be good solvents, and examples of the good solvents include the above-mentioned good solvents. Among them, 2-propanol is preferably included. The temperature for precipitating crystals includes preferably 10°C to 30°C.

The amounts of seed crystals used in the preparation of the Type 1 crystal of Compound (1) is preferably within a range of 0.001 to 10 % by weight, more preferably within a range of 0.005 to 1 % by weight, relative to the Compound (1).

The Type 1 crystal of Compound (1) can be mixed or combined with one or more ingredients selected from the group consisting of the following Group (a), Group (b), Group (c) and Group (d) (hereinafter, referred to as "Present ingredient").

The above-described defined mixing or combining represents that the Type 1 crystal and Present ingredient are used concurrently, separately, or at an interval.

When the Type 1 crystal of Compound (1) and Present ingredient are concurrently used, the Type 1 crystal of Compound (1) and Present ingredient may be incorporated as a separate formulation or one formulation.

One aspect of the present invention relates to a composition comprising one or more ingredients selected from the group consisting of the Group (a), the Group (b), the Group (c) and the Group (d) (that is, Present ingredient) as well as the Type 1 crystal of Compound (1) (hereinafter, the composition is referred to as "Composition A").

The Group (a) represents acetylcholinesterase inhibitors (for example, carbamate insecticides and organophosphate insecticides), GABA-gated chloride ion channel blockers (for example, phenylpyrazole insecticides), sodium channel modulators (for example, pyrethroid insecticides), nicotinic acetylcholine receptor antagonist modulators (for example, neonicotinoid insecticides), nicotinic acetylcholine receptor allosteric modulators, glutamate-gated chloride ion channel allosteric modulators (for example, macrolide insecticides), juvenile hormone mimics, multisite inhibitors, chordotonal organ TRPV channel modulators, mites growth regulators, microbial disruptors of insect midgut membranes, mitochondrial ATP synthase inhibitors, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, inhibitors of mitochondrial electron transport chain complex I, II, III, and IV, voltage-dependent sodium channel blockers, Inhibitors of acetyl CoA carboxylase, ryanodine receptor modulators (for example, diamide insecticides), chordotonal organ modulators, microbial insecticides, as well as other insecticidal active ingredients, miticidal active ingredients and nematicidal active ingredients. These agents are described in the classification based on the IRAC mode of action.

The Group (b) is a group consisting of nucleic acid synthesis inhibitors (for example, phenylamide fungicides and acylamino acid fungicides), cytostatic and cytoskeletal inhibitors (for example, MBC fungicides), respiration inhibitors (for example, QoI fungicides and QiI fungicides), amino-acid synthesis and protein synthesis inhibitors (for example, anilinopyridine fungicides), signal-transduction inhibitors, lipid synthesis and membrane synthesis inhibitors, sterol biosynthesis inhibitors (for example, DMI fungicides such as triazoles), cell wall synthesis inhibitors, melanin synthesis inhibitors, plant defense inducer, multisite fungicides, microbial fungicides, and other fungicidal active ingredients. These agents are described in the classification based on the FRAC mode of action.

The Group (c) represents a group of plant growth modulating ingredients (including mycorrhizal fungus and rhizobia).

The Group (d) represents a group of repellent active ingredients consisting of bird repellent ingredients and insect repellent ingredients.

Examples of combinations of Present ingredient and the Type 1 crystal of Compound (1) are recited as follows. For example, the "alanycarb + SX" indicates a combination of alanycarb and SX.

The abbreviation "SX" means the Type 1 crystal of Compound (1). Further, all Present ingredients as described below are known ingredients and can be obtained from commercially available formulations or prepared by known methods. When Present ingredient represents a microorganism, the Present ingredient can be obtained from a microorganism depositary authority. The number in parentheses represents CAS RN (registered trademark).

A combination of Present ingredient in the above-mentioned Group (a) and the Type 1 crystal of Compound (1):
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, Cbark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC (2-methyl-4,6-dinitrophenol) + SX, doramectin + SX, dried leaves of Dryopteris filix-mas + SX, emamectin-benzoate + SX, empenthrin + SX, endosulfan + SX, EPN (O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin) + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050.20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (217109909-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein CrylAc + SX, BBT crop protein Cry1Fa + SX, BT crop protein Cry1A.105 + SX, BBT crop protein Cry2Ab + SX, BT crop protein Vip3A + SX, BT crop protein mCry3A + SX, BT crop protein Cry3Ab + SX, BBT crop protein Cry3Bb + SX, BT crop protein Cry34Ab1/Cry35Ab1 + SX, Adoxophyes orana GV(granulovirus) strain BV-00.01 + SX, Anticarsia gemmatalis MNPV(multiple nucleocapsid nucleopolyhedrovirus) + SX, Autographa californica MNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX,
Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-18.57 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL00.2 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

A combination of Present ingredient in the above-mentioned Group (b) and the Type 1 crystal of Compound (1):
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper (II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX,
fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX,
pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (12.0.2781-91-6) + SX, N'-{4-[(4,5-dichlorothiazol-2-yl)oxy]-2,5-dimethylphenyl}-N-ethyl-N-methylmethanimidamide (929908.57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-0.2-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660.27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-0.2-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (20.98918-25-1) + SX, 5,5-dimethyl-2-({4-[5-(trifluoromethyl)-1,2,4-oxadiazol-3-yl]phenyl}methyl)isoxazolidin-3-one (20.98918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-S-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl) (2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{ [(oxolan-3-yl) carbonyl] (2, 6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl] -2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K10.26 + SX, Agrobacterium radiobactor strain K84 + SX, Bacillus amyloliquefaciens strain PTA-4838 (Aveo(Trade Mark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB10.2 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF28.56 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST3000.2/AQ3000.2 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX,
Pseudomonas fluorescens strain CL145A + SX, Pseudomonas fluorescens strain G7090 + SX, Pseudomonas sp. strain CAB-0.2 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM-1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SXₒ

A combination of Present ingredient in the above-mentioned Group (c) and the Type 1 crystal of Compound (1): 1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyric acid + SX, methyl 5-(trifluoromethyl)benzo[b]thiophen-2-carboxylate + SX, 3-[(6-chloro-4-phenylquinazolin-2-yl)amino]propane-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY20.9 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

A combination of Present ingredient in the above-mentioned Group (d) and the Type 1 crystal of Compound (1):
anthraquinone + SX, deet + SX, icaridin + SX.

The ratio of the Type 1 crystal of Compound (1) to Present ingredient is not limited unless otherwise indicated, and include for example, as a ratio by weight (Type 1 crystal of Compound (1): Present ingredient) 1000:1 to 1:1000, 500:1 to 1:500, 100:1 to 1:100, 50:1, 20:1, 10:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, 1:50, and the like.

The Type 1 crystal of Compound (1) can control harmful arthropods (such as harmful insects, harmful mites and the like), harmful mollusks and harmful nematodes. Examples of harmful arthropods, harmful mollusks and harmful nematodes include the followings.

### Hemiptera :

from the family Delphacidae, small brown planthopper (Laodelphax striatellus), brown planthopper (Nilaparvata lugens), white-backed planthopper (Sogatella furcifera), corn planthopper (Peregrinus maidis), cereal leafhopper (Javesella pellucida), sugarcane leafhopper (Perkinsiella saccharicida), Tagosodes orizicolus, Stenocranus pacificus, and the like;
from the family Cicadellidae, green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), Amrasca biguttula biguttula, and the like;
from the family Aphrophoridae, meadow spittlebug (Philaenus spumarius), and the like;
from the family Cercopidae, Mahanarva posticata, Mahanarva fimbriolata, and the like;
from the family Aphididae, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid (Dysaphis plantaginea), false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), English grain aphid (Sitobion avenae), and the like;
from the family Phylloxeridae, grapevine phylloxera (Daktulosphaira vitifoliae), Pecan phylloxera (Phylloxera devastatrix), Pecan leaf phylloxera (Phylloxera notabilis), Southern pecan leaf phylloxera (Phylloxera russellae), and the like;
from the family Adelgidae, hemlock woolly aphid (Adelges tsugae), Balsam woolly adelgid (Adelges piceae), Cholodkovsky (Aphrastasia pectinatae), and the like;
from the family Pentatomidae, black rice bug (Scotinophara lurida), Malayan rice black bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), purple stink bug with white stars (Eysarcoris annamita), brown marmorated stink bug (Halyomorpha halys), green plant bug (Nezara viridula), Brown stink bug (Euschistus heros), Red banded stink bug (Piezodorus guildinii), Oebalus pugnax, Dichelops melacanthus, legume stink bug (Piezodorus hybneri) and the like;
from the family Cydnidae, Burrower brown bug (Scaptocoris castanea);
from the family Alydidae, bean bug (Riptortus pedestris), corbett rice bug (Leptocorisa chinensis), rice bug (Leptocorisa acuta), and the like;
from the family Coreidae, rice stinkbug (Cletus punctiger), Australian leaf-footed bug (Leptoglossus australis), and the like;
from the family Lygaeidae, oriental chinch bug (Caverelius saccharivorus), seed bug (Togo hemipterus), chinch bug (Blissus leucopterus), and the like;
from the family Miridae, rice leaf bug (Trigonotylus caelestialium), sorghum plant bug (Stenotus rubrovittatus), wheat leaf bug (Stenodema calcarata), American tarnished plant bug (Lygus lineolaris), and the like;
from the family Aleyrodidae, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), Pealius euryae, and the like;
from the family Diaspididae, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), citrus snow scale (Unaspis citri), and the like;
from the family Coccidae, pink wax scale (Ceroplastes rubens) ;
from the family Margarodidae, fluted scale (Icerya purchasi), seychelles fluted scale (Icerya seychellarum), and the like;
from the family Pseudococcidae, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), tuttle mealybug (Brevennia rehi), and the like;
from the family Psyllidae, citrus psylla (Diaphorina citri), two-spotted citrus psyllid (Trioza erytreae), pear sucker (Cacopsylla pyrisuga), Cacopsylla chinensis, potato psyllid (Bactericera cockerelli), Pear psylla (Cacopsylla pyricola), and the like;
from the family Tingidae, sycamore lace bug (Corythucha ciliata), aster tingid (Corythucha marmorata), Japanese pear lace bug (Stephanitis nashi), azalea lace bug (Stephanitis pyrioides), and the like;
from the family Cimicidae, common bed bug (Cimex lectularius), tropical bed bug (Cimex hemipterus), and the like;
from the family Cicadidae, Giant Cicada (Quesada gigas), and the like;
from the family Reduviidae, Triatoma infestans, Triatoma rubrofasciata, Triatoma dimidiate, Rhodonius prolixus, and the like.

### Lepidoptera:

from the family Crambidae, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Pediasia teterrellus), rice case-worm (Nymphula depunctalis), Sugarcane borer (Diatraea saccharalis), Eggplant fruit and shoot borer (Leucinodes orbonalis) and the like;
from the family Pyralidae, lesser cornstalk borer (Elasmopalpus lignosellus), mealworm moth (Plodia interpunctella), persimmon bark borer (Euzophera batangensis), fig moth (Cadra cautella), and the like;
from the family Noctuidae, common cutworm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), Spodoptera cosmioides, Southern armyworm (Spodoptera eridania), black cutworm (Agrotis ipsilon), turnip moth (Agrotis segetum), beet worm (Autographa nigrisigna), rice looper (Plusia festucae), soybean looper (Chrysodeixis includens), Trichoplusia spp., Heliothis spp.(such as tobacco budworm (Heliothis virescens)), Helicoverpa spp. (such as tobacco budworm (Helicoverpa armigera), corn earworm (Helicoverpa zea)), Velvetbean caterpillar (Anticarsia gemmatalis), Cotton leafworm (Alabama argillacea), Hop vine borer (Hydraecia immanis), and the like;
from the family Pieridae, common cabbage worm (Pieris rapae), and the like;
from the family Tortricidae, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), Bean Shoot Borer (Epinotia aporema), citrus fruit borer (Citripestis sagittiferella), European grapevine moth (Lobesia botrana), and the like;
from the family Gracillariidae, tea leaf roller (Caloptilia theivora), Asiatic apple leaf miner (Phyllonorycter ringoniella), and the like;
from the family Carposinidae, peach fruit moth (Carposina sasakii), and the like;
from the family Lyonetiidae, Coffee Leaf miner (Leucoptera coffeella), peach leaf miner (Lyonetia clerkella), Lyonetia prunifoliella, and the like;
from the family Lymantriidae, Lymantria spp. (such as gypsy moth (Lymantria dispar)), Euproctis spp. (such as tea lymantriid (Euproctis pseudoconspersa)), and the like;
from the family Plutellidae, diamondback moth (Plutella xylostella), and the like;
from the family Gelechiidae, peach worm (Anarsia lineatella), sweetpotato leaf folder (Helcystogramma triannulella), pink bollworm (Pectinophora gossypiella), potato moth (Phthorimaea operculella), Tuta absoluta, and the like;
from the family Arctiidae, American white moth (Hyphantria cunea), and the like;
from the family Castniidae, Giant Sugarcane borer (Telchin licus), and the like;
from the family Cossidae, Cossus insularis, and the like;
from the family Geometridae, Ascotis selenaria, and the like;
from the family Limacodidae, blue-striped nettle grub (Parasa lepida), and the like;
from the family Stathmopodidae, persimmon fruit moth (Stathmopoda masinissa), and the like;
from the family Sphingidae, tobacco hornworm (Acherontia lachesis), and the like;
from the family Sesiidae, Nokona feralis, cherry borer (Synanthedon hector), Synanthedon tenuis, and the like:
   from the family Hesperiidae, rice skipper (Parnara guttata), and the like;
   from the family Tineidae, casemaking clothes moth (Tinea translucens), common clothes moth (Tineola bisselliella), and the like.

### Thysanoptera:

from the family Thripidae, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, avocado thrips (Scirtothrips perseae), and the like;
from the family Phlaeothripidae, aculeated rice thrips (Haplothrips aculeatus), and the like.

### Diptera:

from the family Anthomyiidae, seedcorn maggot (Delia platura), onion maggot (Delia antiqua), beet leaf miner (Pegomya cunicularia), and the like;
from the family Ulidiidae, sugarbeet root maggot (Tetanops myopaeformis), and the like;
from the family Agromyzidae, rice leaf miner (Agromyza oryzae), tomato leaf miner (Liriomyza sativae), chrysanthemum leaf miner (Liriomyza trifolii), pea leafminer (Chromatomyia horticola), and the like;
from the family Chloropidae, rice stem maggot (Chlorops oryzae), and the like;
from the family Tephritidae, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), Japanese cherry fruit fly (Rhacochlaena japonica), and the like;
from the family Ephydridae, smaller rice leaf miner (Hydrellia griseola), whorl maggot (Hydrellia philippina), paddy stem maggot (Hydrellia sasakii), and the like;
from the family Drosophilidae, cherry drosophila (Drosophila suzukii), Drosophila melanogaster, and the like;
from the family Phoridae, Megaselia spiracularis, and the like;
from the family Psychodidae, Clogmia albipunctata, and the like;
from the family Sciaridae, Bradysia difformis, and the like;
from the family Sciaridae, Bradysia difformis, and the like;
from the family Cecidomyiidae, hessian fly (Mayetiola destructor), paddy gall fly (Orseolia oryzae), and the like;
from the family Diopsidae, Diopsis macrophthalma, and the like;
from the family Glossinidae, Glossina palpalis, Glossina morsitans, and the like;
from the family Simuliidae, Simulium japonicum, Simulium damnosum, and the like;
from the family Phlebotominae;
from the family Tipulidae, rice crane fly (Tipula aino), Common cranefly (Tipula oleracea), European cranefly (Tipula paludosa), and the like;
from the family Culicidae, common house mosquito (Culex pipiens pallens), Culex tritaeniorhynchus, Culex pipiens f. molestus, southern house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, Anopheles minimus, and the like;
from the family Simulidae, Prosimulium yezoensis, Simulium ornatum, and the like;
from the family Tabanidae, Tabanus trigonus, and the like;
from the family Muscidae, house fly (Musca domestica), false stable fly (Muscina stabulans), biting house fly (Stomoxys calcitrans), buffalo fly (Haematobia irritans) , and the like;
from the family Calliphoridae;
from the family Sarcophagidae;
from the family Chironomidae, Chironomus plumosus, Chironomus yoshimatsui, Glyptotendipes tokunagai, and the like;
from the family Fannidae.

### Coleoptera:

from the family Chrysomelidae, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), Cucurbit Beetle (Diabrotica speciosa), and the like); bean leaf beetle (Cerotoma trifurcata), barley leaf beetle (Oulema melanopus), cucurbit leaf beetle (Aulacophora femoralis), striped flea beetle (Phyllotreta striolata), Cabbage flea beetle (Phyllotreta cruciferae), Western black flea beetle (Phyllotreta pusilla), Cabbage stem flea beetle (Psylliodes chrysocephala), hop Flea Beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, tobacco flea beetle (Epitrix hirtipennis), radish flea beetle (Phaedon brassicae), Medythia nigrobilineata, and the like;
from the family Carabidae, Seedcorn beetle (Stenolophus lecontei), Slender seedcorn beetle (Clivina impressifrons), and the like;
from the family Scarabaeidae, cupreus chafer (Anomala cuprea), soybean beetle (Anomala rufocuprea), Anomala albopilosa, Japanese beetle (Popillia japonica), yellowish elongate chafer (Heptophylla picea), European Chafer (Rhizotrogus majalis), Tomarus gibbosus, Holotrichia spp., Phyllophaga spp. (such as June beetle (Phyllophaga crinita)), and Diloboderus spp. (such as Diloboderus abderus), and the like;
from the family Anthriibidae, coffee bean weevil (Araecerus coffeae), and the like;
from the family Aponidae, sweet potato weevil (Cylas formicarius)), and the like;
from the family Bruchidae, Mexican bean weevil (Zabrotes subfasciatus), and the like;
from the family Scolytidae, pine beetle (Tomicus piniperda), Coffee Berry Borer (Hypothenemus hampei), and the like;
from the family Curculionidae, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineatocollis, boll weevil (Anthonomus grandis), nunting billbug (Sphenophorus venatus), Southern Corn Billbug (Sphenophorus callosus), Soybean stalk weevil (Sternechus subsignatus), Sugarcane weevil (Sphenophorus levis), rusty gourd-shaped weevil (Scepticus griseus), brown gourd-shaped weevil (Scepticus uniformis), Aracanthus spp. (such as Aracanthus mourei), cotton root borer (Eutinobothrus brasiliensis), and the like;
from the family Tenebrionidae, red meal beetle (Tribolium castaneum), mason beetle (Tribolium confusum), lesser mealworm (Alphitobius diaperinus), and the like;
from the family Coccinellidae, twenty-eight-spotted ladybird (Epilachna vigintioctopunctata), and the like;
from the family Bostrychidae, common powder-post beetle (Lyctus brunneus), lesser grain borer (Rhizopertha dominica), and the like;
from the family Ptinidae;
from the family Cerambycidae, citrus long-horned beetle (Anoplophora malasiaca), Migdolus fryanus, Aromia bungii, and the like;
from the family Elateridae, Melanotus okinawensis, barley wireworm (Agriotes fuscicollis), Melanotus legatus, Anchastus spp., Conoderus spp., Ctenicera spp., Limonius spp., Aeolus spp., and the like;
from the family Staphylinidae, Paederus fuscipes, and the like;
from the family Dermestidae, varied carpet beetle (Anthrenus verbasci), hide beetle (Dermestes maculates), khapra beetle (Trogoderma granarium), and the like;
from the family Anobidae, tobacco beetle (Lasioderma serricorne) and biscuit beetle (Stegobium paniceum), and the like;
from the family Laemophloeidae, flat grain beetle (Cryptolestes ferrugineus), and the like;
from the family Silvanidae, saw-toothed grain beetle (Oryzaephilus surinamensis), and the like;
from the family Nitidulidae, pollen beetle (Brassicogethes aeneu), and the like.

### Orthoptera:

from the family Acrididae, oriental migratory locust (Locusta migratoria), Moroccan locust (Dociostaurus maroccanus), Australian plague locust (Chortoicetes terminifera), red locust (Nomadacris septemfasciata), Brown Locust (Locustana pardalina), Tree Locust (Anacridium melanorhodon), Italian Locust (Calliptamus italicus), Differential grasshopper (Melanoplus differentialis), Two striped grasshopper (Melanoplus bivittatus), Migratory grasshopper (Melanoplus sanguinipes), Red-Legged grasshopper (Melanoplus femurrubrum), Clearwinged grasshopper (Camnula pellucida), desert locust (Schistocerca gregaria), Yellow-winged locust (Gastrimargus musicus), Spur-throated locust (Austracris guttulosa) , Japanese grasshopper (Oxya yezoensis), rice grasshopper (Oxya japonica), Bombay locust (Patanga succincta), and the like;
from the family Gryllotalpidae, oriental mole cricket (Gryllotalpa orientalis), and the like;
from the family Gryllidae, house cricket (Acheta domestica), emma field cricket (Teleogryllus emma), and the like;
from the family Tettigoniidae, Mormon cricket (Anabrus simplex), and the like.

### Hymenoptera:

from the family Tenthredinidae, beet sawfly (Athalia rosae), nippon cabbage sawfly (Athalia japonica), and the like;
from the family Formicidae, Solenopsis spp. (such as red imported fire ant (Solenopsis invicta), tropical fire ant (Solenopsis geminata)), Atta spp. (such as Brown leaf-cutting ant (Atta capiguara)), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), long-legged ant (Anoplolepis gracilipes), and the like;
from the family Vespidae, Asian giant hornet (Vespa mandarinia), Vespa simillima, Vespa analis, Asian hornet (Vespa velutina), Polistes jokahamae, and the like;
from the family Siricidae, pine wood wasp (Urocerus gigas), and the like;
from the family Bethylidae.

### Blattodea:

from the family Blattellidae, German cockroach (Blattella germanica), and the like;
from the family Blattidae, smoky-brown cockroach (Periplaneta fuliginosa), American cockroach (Periplaneta americana), Australian cockroach (Periplaneta australasiae), brown cockroach (Periplaneta brunnea), black cockroach (Blatta orientalis), and the like;
from the family Termitidae, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, Cornitermes cumulans, and the like.

### Siphonaptera:

from the family Pulicidae, human flea (Pulex irritans), cat flea (Ctenocephalides felis), dog flea (Ctenocephalides canis), oriental rat flea (Xenopsylla cheopis), chicken flea (Echidnophaga gallinacea), and the like;
from the family Hectopsyllidae, Tunga penetrans, and the like;
from the family Ceratophyllidae, Northern rat fleas (Nosopsyllus fasciatus), and the like.

### Psocodae:

from the family Pediculidae, head louse (Pediculus humanus capitis)), and the like;
from the family Pthiridae, crab louse (Pthirus pubis), and the like;
from the family Haematopinidae, short-nosed cattle louse (Haematopinus eurysternus, pig louse (Haematopinus suis), and the like;
from the family Linognathidae, blue cattle louse (Linognathus vituli), sheep face louse (Linognathus ovillus), capillate louse (Solenopotes capillatus), and the like;
from the family Bovicoliidae, cattle biting louse (Bovicola bovis), sheep body louse (Bovicola ovis), Bovicola breviceps, Damalinia forficula, Werneckiella spp., and the like;
from the family Trichodectidae, dog biting louse (Trichodectes canis), cat louse (Felicola subrostratus), and the like;
from the family Menoponidae, common chicken louse (Menopon gallinae, chicken body louse (Menacanthus stramineus), Trinoton spp., and the like,
from the family Trimenoponidae, Cummingsia spp., and the like;
from the family Trogiidae, larger pale booklouse (Trogium pulsatorium), and the like;
from the family Liposcelidae or Liposcelididae, Liposcelis corrodens, Liposcelis bostrychophila, Liposcelis pearmani, Liposcelis entomophila, and the like.

Thysanura:
from the family Lepismatidae, oriental silverfish (Ctenolepisma villosa), moth fish (Lepisma saccharina), and the like.

### Acari:

from the family Tetranychidae, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), Oligonychus spp., and the like;
from the family Eriophyidae, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, Shevtchenkella sp., and the like;
from the family Tarsonemidae, broad mite (Polyphagotarsonemus latus), and the like;
from the family Tenuipalpidae, Brevipalpus phoenicis, and the like;
from the family Tuckerellidae;
from the family Ixodidae, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), Dermacentor reticulatus, Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum)), Rhipicephalus microplus, Boophilus annulatus, brown dog tick (Rhipicephalus sanguineus), Rhipicephalus appendiculatus, Rhipicephalus decoloratus, and the like;
from the family Argasidae, fowl tick (Argas persicus), Ornithodoros hermsi, Ornithodoros turicata, and the like;
from the family Acaridae, cereal mite (Tyrophagus putrescentiae), grassland mite (Tyrophagus similis), and the like;
from the family Pyroglyphidae, American house dust mite (Dermatophagoides farinae), European house dust mite (Dermatophagoides pteronyssinus), and the like;
from the family Cheyletidae, Cheyletus eruditus, Cheyletus malaccensis, Chelacaropsis moorei, Cheyletiella yasguri, and the like;
from the family Psoroptidae, sheep scab mite (Psoroptes ovis), horse psoroptic mange mite (Psoroptes equi), Knemidocoptes mutans, ear mange mite (Otodectes cynotis), Chorioptes spp., and the like;
from the family Sarcoptidae, Notoedres cati, Notoedres muris, itch mite (Sarcoptes scabiei), and the like;
from the family Listrophoridae, Listrophorus gibbus, and the like;
from the family Dermanyssidae, bird mite (Dermanyssus gallinae), and the like;
from the family Macronyssidae, feather mite (Ornithonyssus sylviarum), tropical rat mite (Ornithonyssus bacoti), and the like;
from the family Varroidae, Varroa jacobsoni, and the like;
from the family Demodicidae, dog follicle mite (Demodex canis), cat follicle mite (Demodex cati), and the like;
from the family Trombiculidae, Leptotrombidium akamushi, Leptotrombidium pallidum, Leptotrombidium scutellare, and the like.

### Araneae:

from the family Eutichuridae, Cheiracanthium japonicum, and the like;
from the family Theridiidae, red-back spider (Latrodectus hasseltii), and the like.

### Polydesmida:

from the family Paradoxosomatidae, flat-backed millipede (Oxidus gracilis), Nedyopus tambanus, and the like.

### Isopoda:

from the family Armadillidiidae, common pill bug (Armadillidium vulgare), and the like.

### Chilopoda:

from the family Scutigeridae, Thereuonema hilgendorfi, and the like.
from the family Scolopendridae, giant tropical centipede (Scolopendra subspinipes), and the like;
from the family Ethopolyidae, Bothropolys rugosus, and the like.

### Gastropoda:

from the family Limacidae, tree slug (Limax marginatus), garden tawny slug (Limax flavus), and the like;
from the family Philomycidae, Meghimatium bilineatum, and the like;
from the family Ampullariidae, golden apple snail (Pomacea canaliculata), and the like;
from the family Lymnaeidae, Austropeplea ollula, and the like.

### Nematoda:

from the family Aphelenchoididae, rice white-tip nematode (Aphelenchoides besseyi), and the like;
from the family Pratylenchidae, root lesion nematode (Pratylenchus coffeae), Pratylenchus brachyurus, California meadow nematode (Pratylenchus neglectus), Radopholus similis, and the like;
from the family Heteroderidae, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), white potato cyst nematode (Globodera pallida), colombia root-knot nematode (Meloidogyne chitwoodi), and the like;
from the family Hoplolaimidae, Rotylenchulus reniformis, and the like;
from the family Anguinidae, strawberry bud nematode (Nothotylenchus acris), stem nematode (Ditylenchus dipsaci), and the like;
from the family Tylenchulidae, citrus nematode (Tylenchulus semipenetrans), and the like;
from the family Longidoridae, dagger nematode (Xiphinema index), and the like;
from the family Trichodoridae;
from the family Parasitaphelenchidae, pine wilt disease (Bursaphelenchus xylophilus), and the like.

The harmful arthropods such as harmful insects and harmful mites, harmful mollusks and harmful nematodes may be those having a reduced susceptibility to or a developed resistance to an insecticide, a miticide, a molluscicide or a nematicide.

The method for controlling harmful arthropods of the present invention is conducted by applying an effective amount of the Type 1 crystal of Compound (1) or the Composition A to a harmful arthropod directly and/or a habitat where the harmful arthropod lives (for example, plant, soil, an interior of a house, and animal). Examples of a method for controlling harmful arthropods of the present invention include foliar application, soil application, root application, shower application, smoking application, water-surface application, and seed application.

The Type 1 crystal of Compound (1) or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the Type 1 crystal of Compound (1) or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the Type 1 crystal of Compound (1). The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the Type 1 crystal of Compound (1) or the Composition A to soils include a method of applying an effective amount of the Type 1 crystal of Compound (1) or the Composition A to soils before planting plants or after planting plants, a method of applying an effective amount of the Type 1 crystal of Compound (1) or the Composition A to a root part of a crop to be protected from damage such as ingestion by harmful arthropods, and a method of controlling harmful arthropods that ingest a plant by permeating and transferring an effective amount of the Type 1 crystal of Compound (1) or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Type 1 crystal of Compound (1) or the Composition A to seeds or vegetative reproductive organs, and specific examples thereof include spraying treatment in which a suspension of the Type 1 crystal of Compound (1) or the Composition A is sprayed onto seed surface or the vegetative reproductive organ surface in the form of mist; smearing treatment in which the Type 1 crystal of Compound (1) or the Composition A is coated a surface of seeds or the vegetative reproductive organ; a soaking treatment in which the seeds are soaked into the solution of the Type 1 crystal of Compound (1) or the Composition A for a certain time; and a method for coating the seeds or the vegetative reproductive organ with a carrier containing the Type 1 crystal of Compound (1) or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for exmaple, a method in which the formulations comprising as an active component the Type 1 crystal of Compound (1) only are applied, and seeds or vegetative reproductive organs are air dried, followed by applying the formulations comprising Present ingredient: and a method in which the formulations comprising as an active component the Type 1 crystal of Compound (1) and Present ingredients are applied, and seeds or vegetative reproductive organs are air dried, followed by applying the formulations comprising Present ingredients other than the already-applied Present ingredients, are included.

As used herein, seeds or vegetative reproductive organs carrying the Type 1 crystal of Compound (1) or the Composition A means seeds or vegetative reproductive organs in the state where the Type 1 crystal of Compound (1) or the Composition A is adhered to a surface of the seeds or the vegetative reproductive organ. The above-described seeds or vegetative reproductive organs carrying the Type 1 crystal of Compound (1) or the Composition A may be adhered by any other materials that are different from the Type 1 crystal of Compound (1) or the Composition A before or after being adhered the Type 1 crystal of Compound (1) or the Composition A to the seeds or vegetative reproductive organs.

Also, when the Composition A is adhered in a form of layer(s) to a surface of seeds or vegetative reproductive organ, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the Type 1 crystal of Compound (1) or the Composition A can be obtained, for example, by applying the formulations comprising the Type 1 crystal of Compound (1) or the Composition A by the above-described application method to seeds to seeds or vegetative reproductive organs.

When the Type 1 crystal of Compound (1) or the Composition A is applied for controlling pests in agricultural fields, the application dose thereof is usually within a range of 1 to 10,000 g of the Type 1 crystal of Compound (1) per 10,000 m². In the case of being applied to seeds or vegetative reproductive organs, the dose of application dose thereof is usually within a range of 0.001 to 100 g of the Type 1 crystal of Compound (1) per 1 Kg of seeds or vegetative reproductive organs. When the Type 1 crystal of Compound (1) or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, with respect to the Type 1 crystal of Compound (1) or the Composition A, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Type 1 crystal of Compound (1) or the Composition A is used to control harmful arthropods that live inside a house, the application dose as an amount of the Type 1 crystal of Compound (1) is usually within a range from 0.01 to 1,000 mg per 1 m² of an area to be treated, in the case of using it on a planar area. In the case of using it spatially, the application dose as an amount of the Type 1 crystal of Compound (1) is usually within a range from 0.01 to 500 mg per 1 m³ of the space to be treated. When the Type 1 crystal of Compound (1) or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Type 1 crystal of Compound (1) or the Composition A is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the Type 1 crystal of Compound (1) the Composition A is usually within a range from 0.1 to 1,000 mg per 1 kg of an animal body weight.

Also, the Type 1 crystal of Compound (1) or the Composition A may be used as an agent for controlling harmful arthropods in agricultural lands such as paddy fields, fields, turfs, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is explained in more detail by Preparation Examples, Formulation Examples, and Test Examples, however, the present invention should not be limited to only these Examples.

The measurement condition of a powder X-ray diffraction, the measurement condition of FT-IR, and the measurement condition of melting point are as follows.

### (Measurement condition of a powder x-ray diffraction)

Powder X-ray diffraction device: XtaLAB Synergy-i (manufactured by Rigaku Corporation)
X-ray output: Cu-Kα, 50 kV, 40 mA
Step width: 0.01°
Scan width: 0° to 61.72°

### (Measurement condition of a single crystal X-ray diffraction)

Powder X-ray diffraction device: XtaLAB Synergy-i (manufactured by Rigaku Corporation)
Detector: HPC detector (HyPix-Bantam)
X-ray output: Cu-Kα (λ=1.5418Å), 50 kV, 40 mA
Measurement temperature: 100K
Structural analysis: ShelXT (Sheldrick, G.M. (2015). Acta Cryst. A71, 3-8)
Refinement: olex2.refine (Bourhis, L.J., Dolomanov, O.V., Gildea, R.J., Howard, J.A.K., Puschmann, H. (2015). Acta Cryst. A71, 59-75.)

### (FT-IR measurement condition)

FT-IR Spectroscopy device: IRSpirit (manufactured by SHIMADZU Corporation)
Measurement method: Attenuated Total Reflection
Accumulation counts: 40
Resolution: 4cm⁻¹

### (Melting point measurement condition)

Melting point measurement device: M-565 (manufactured by Nihon BUCHI)
Heating condition: 0.5°C /min

### Reference Example 1

Compound (1) was prepared from 5-cyclopropyl-3-(ethylsulfonyl)-N-(2-hydroxy-5-((trifluoromethyl)sulfonyl)phenyl)picoline amide according to a process described in Reference Example 4 of Patent Literature 3. The melting point of the obtained Compound (1) was 175 to 178°C.

### Example 1

2-Propanol (140 mL) was added to the Compound (1) obtained in the above-mentioned Reference Example 1 (1 g), and the mixture was heated to 60°C to dissolve it completely. The mixture was allowed to stand at room temperature, and solids were collected by filtering the resulting mixture to obtain the Type 1 crystal of Compound (1).

As the result of measuring a powder X-ray diffraction with Cu-Kα radiation of the obtained Type 1 crystal of Compound (1), the diffraction angles (2θ) of the obtained diffraction peaks were shown in Figure 1. Typical examples of specific diffraction peaks include 14.3°, 15.6°, 17.1°, 18.7°, 20.1°, 22.4°, 23.3°, 24.8°, 26.1° and 28.5°, which are not limited thereto. The diffraction angle (2θ) and integrated intensity (cps°) of the obtained diffraction peaks are shown in Table 1A. As the result of the FT-IR measurement, the peaks as indicated in Figure 2 were shown. Typical examples of specific peaks include 1370 cm⁻¹, 1317 cm⁻¹, 1190 cm⁻¹, 1124 cm⁻¹, 1093 cm⁻¹, 1047 cm⁻¹, 1034 cm⁻¹, 846 cm⁻¹, 826 cm⁻¹, 776 cm⁻¹, 738 cm⁻¹ and 719 cm⁻¹, which are not limited thereto. Also, as the result of measuring a melting point, it was 180 to 181.3°C.

As the result of measuring a single crystal X-ray diffraction of the obtained Type 1 crystal, the crystallographic data of the obtained single crystal was shown in Table 1B.

**Table 1A**

| 2θ | Integrated intensity (cps) |
|---|---|
| 7.40 | 12.1 |
| 14.25 | 62.0 |
| 15.61 | 36.5 |
| 17.14 | 88.3 |
| 18.73 | 107.3 |
| 20.12 | 14.1 |
| 22.44 | 67.9 |
| 23.32 | 36.3 |
| 24.75 | 32.0 |
| 26.07 | 42.7 |
| 28.49 | 29.6 |

**Table 1B**

| Measurement temperature T [K] | 100 |
|---|---|
| Crystal system (class) | Crystal of orthorhombic crystal system (orthorhombic) |
| Space group | P2₁2₁2₁ |
| a [Å] | 6.454 |
| b [Å] | 14.548 |
| c [Å] | 20.855 |
| α [°] | 90 |
| β [°] | 90 |
| γ [°] | 90 |
| Unit cell volume (Volume V[Å³]) | 1957.99 |
| Number of molecules in unit cell (Z) | 4 |
| Density (calculated) [g/cm³] | 1.562 |

### Example 2

Toluene (5 mL) was added to the Compound (1) obtained in the above-mentioned Reference Example 1 (1 g), and the mixture was heated to 60°C to dissolve it completely. The mixture was allowed to stand at room temperature, and solids were collected by filtering the resulting mixture to obtain the Type 1 crystal of Compound (1).

As the result of measuring a powder X-ray diffraction with Cu-Kα radiation of the obtained Type 1 crystal of Compound (1), the diffraction angles (2θ) of the obtained diffraction peaks were shown in Figure 3. Typical examples of specific diffraction peaks include 14.3°, 15.6°, 17.2°, 18.7°, 20.1°, 22.5°, 23.3°, 24.7°, 26.0° and 28.5°, which are not limited thereto. The diffraction angle (2θ) and integrated intensity (cps°) of the obtained diffraction peaks are shown in Table 2A. As the result of the FT-IR measurement, the peaks as indicated in Figure 4 were shown. Typical examples of specific peaks include 1370 cm⁻¹, 1315 cm⁻¹, 1190 cm⁻¹, 1124 cm⁻¹, 1093 cm⁻¹, 1047 cm⁻¹, 1034 cm⁻¹, 846 cm⁻¹, 826 cm⁻¹, 776 cm⁻¹, 738 cm⁻¹ and 719 cm⁻¹, which are not limited thereto. Also, as the result of measuring a melting point, it was 180 to 181.3°C.

**Table 2A**

| 2θ | Integrated intensity (cps) |
|---|---|
| 14.27 | 59.3 |
| 15.61 | 38.6 |
| 17.16 | 78.1 |
| 18.72 | 104.3 |
| 20.11 | 12.9 |
| 22.45 | 65.0 |
| 23.33 | 37.2 |
| 24.70 | 35.5 |
| 26.04 | 46.3 |
| 28.51 | 32.3 |

### Example 3

Tetrahydrofuran (2 mL) was added to the Compound (1) obtained in the above-mentioned Reference Example 1 (0.82 g), and the mixture was heated to 60°C to dissolve it completely. The mixture was allowed to stand at room temperature, and solids were collected by filtering the resulting mixture to obtain the Type 1 crystal of Compound (1).

As the result of measuring a powder X-ray diffraction with Cu-Kα radiation of the obtained Type 1 crystal of Compound (1), the diffraction angles (2θ) of the obtained diffraction peaks were shown in Figure 5. Typical examples of specific diffraction peaks include 14.2°, 15.6°, 17.2°, 18.7°, 20.2°, 22.4°, 23.3°, 24.6°, 26.0° and 28.5°, which are not limited thereto. The diffraction angle (2θ) and integrated intensity (cps°) of the obtained diffraction peaks are shown in Table 3A. As the result of the FT-IR measurement, the peaks as indicated in Figure 6 were shown. Typical examples of specific peaks include 1370 cm⁻¹, 1317 cm⁻¹, 1190 cm⁻¹, 1124 cm⁻¹, 1093 cm⁻¹, 1047 cm⁻¹, 1034 cm⁻¹, 846 cm⁻¹, 826 cm⁻¹, 776 cm⁻¹, 738 cm⁻¹ and 719 cm⁻¹, which are not limited thereto. Also, as the result of measuring a melting point, it was 180 to 181.4°C.

**Table 3A**

| 2θ | Integrated intensity (cps) |
|---|---|
| 7.39 | 15.2 |
| 8.50 | 10.6 |
| 10.52 | 17.6 |
| 14.24 | 67.3 |
| 15.62 | 37.6 |
| 17.16 | 88.1 |
| 18.74 | 114.1 |
| 20.16 | 19.5 |
| 22.41 | 84.7 |
| 23.31 | 36.4 |
| 24.64 | 52.6 |
| 26.03 | 55.8 |
| 28.51 | 39.1 |
| 29.71 | 10.7 |

### Example 4

Tert-butyl methyl ether (60 mL) was added to the Compound (1) obtained in the above-mentioned Reference Example 1 (1.02 g), and the mixture was heated to 60°C to dissolve it completely. The mixture was allowed to stand at room temperature, and solids were collected by filtering the resulting mixture to obtain the Type 1 crystal of Compound (1).

As the result of measuring a powder X-ray diffraction with Cu-Kα radiation of the obtained Type 1 crystal of Compound (1), the obtained diffraction angles (2θ) were shown in Figure 7. Typical examples of specific diffraction peaks include 14.3°, 15.6°, 17.1°, 18.7°, 20.2°, 22.4°, 23.3°, 24.6°, 26.0° and 28.5°, which are not limited thereto. The diffraction angle (2θ) and integrated intensity (cps°) of the obtained diffraction peaks are shown in Table 4A. As the result of the FT-IR measurement, the peaks as indicated in Figure 8 were shown. Typical examples of specific peaks include 1370 cm⁻¹, 1315 cm⁻¹, 1190 cm⁻¹, 1124 cm⁻¹, 1093 cm⁻¹, 1047 cm⁻¹, 1034 cm⁻¹, 846 cm⁻¹, 826 cm⁻¹, 776 cm⁻¹, 738 cm⁻¹ and 719 cm⁻¹, which are not limited thereto. Also, as the result of measuring a melting point, it was 180 to 181.4°C.

**Table 4A**

| 2θ | Integrated intensity (cps) |
|---|---|
| 7.42 | 12.5 |
| 10.53 | 23.5 |
| 14.25 | 59.9 |
| 15.62 | 39.5 |
| 17.14 | 80.8 |
| 18.73 | 103.5 |
| 20.17 | 22.7 |
| 22.40 | 81.8 |
| 23.32 | 35.2 |
| 24.59 | 54.7 |
| 26.02 | 56.9 |
| 28.51 | 38.8 |
| 29.71 | 10.5 |

### Example 5

Acetone (2 mL) was added to the Compound (1) obtained in the above-mentioned Reference Example 1 (0.93 g), and the mixture was heated to 60°C to dissolve it completely. The mixture was allowed to stand at room temperature, and solids were collected by filtering the resulting mixture to obtain the Type 1 crystal of Compound (1).

As the result of measuring a powder X-ray diffraction with Cu-Kα radiation of the obtained Type 1 crystal of Compound (1), the obtained diffraction angles (2θ) were shown in Figure 9. Typical examples of specific diffraction peaks include 14.2°, 15.6°, 17.1°, 18.7°, 20.1°, 22.4°, 23.3°, 24.6°, 26.0° and 28.5°, which are not limited thereto. The diffraction angle (2θ) and integrated intensity (cps°) of the obtained diffraction peaks are shown in Table 5A. Also, as the result of the FT-IR measurement, the peaks as indicated in Figure 10 were shown. Typical examples of specific peaks include 1370 cm⁻¹, 1317 cm⁻¹, 1190 cm⁻¹, 1124 cm⁻¹, 1093 cm⁻¹, 1047 cm⁻¹, 1034 cm⁻¹, 846 cm⁻¹, 826 cm⁻¹, 776 cm⁻¹, 738 cm⁻¹ and 719 cm⁻¹, which are not limited thereto. Also, as the result of measuring a melting point, it was 180 to 181.4°C.

**Table 5A**

| 2θ | Integrated intensity (cps) |
|---|---|
| 7.42 | 13.6 |
| 10.45 | 13.3 |
| 14.23 | 64.9 |
| 15.61 | 36.4 |
| 17.13 | 90.9 |
| 18.71 | 116.0 |
| 20.07 | 19.6 |
| 22.39 | 85.1 |
| 23.29 | 40.1 |
| 24.57 | 52.8 |
| 26.01 | 60.6 |
| 28.46 | 41.1 |
| 29.66 | 13.4 |

### Reference Example 1

### Obtaining amorphous of Compound (1)

Acetonitrile (5 mL) was added to the Compound (1) obtained in the above-mentioned Reference Example 1 (1.24 g) and the mixture was heated to 60°C to dissolve it completely. The mixture was allowed to stand at room temperature, and solids were collected by filtering the resulting mixture to obtain the Type 1 crystal of Compound (1) .As the result of measuring a powder X-ray diffraction with Cu-Kα radiation on the obtained solids, no X-ray diffraction peak was confirmed, which was found that the solids were amorphous.

Next, the formulation examples of Compound (1) of the present invention are shown below. In the formulation examples, the "parts" represents "part by weight" unless otherwise specified.

### Formulation Example 1

Thirty-five (35) parts of a mixture of ammonium polyoxyethylene alkyl ether sulfate and wet silica (weight ratio: 1:1), 10 parts of the Type 1 crystal of Compound (1), and 55 parts of water are mixed, and the mixture is then finely-ground by a wet grinding method to obtain a formulation.

### Formulation Example 2

Fifty (50) parts of the Type 1 crystal of Compound (1), 3 parts of calcium lignin sulfonate, 2 parts of sodium lauryl sulfate, and 45 parts of silica are well mixed-grinding to obtain a formulation.

### Formulation Example 3

Five (5) parts of the Type 1 crystal of Compound (1), 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

### Formulation Example 4

Two (2) parts of the Type 1 crystal of Compound (1), 1 part of silica, 2 parts of calcium lignin sulfonate, 30 parts of bentonite and 65 parts of kaolin clay are mixed-grinding, and thereto is added an appropriate amount of water, and the mixture is well kneaded and is then granulated with a granulator and dried to obtain a formulation.

### Formulation Example 5

Ten (10) parts of the Type 1 crystal of Compound (1) is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

### Formulation Example 6

Zero point one (0.1) parts of the Type 1 crystal of Compound (1) and 39.9 parts of kerosene are dissolved while mixing, and the mixture is placed in an aerosol container, and 60 parts of liquefied petroleum gas (mixture of propane, butane and isobutane; saturated vapor pressure: 0.47 MPa (25°C)) is filled in the container to obtain a formulation.

### Formulation Example 7

Zero point two (0.2) parts of the Type 1 crystal of Compound (1), 50 parts of pyrethrum extract dreg powder, 30 parts of Machilus thunbergii powder, and 19.8 parts of wood powder are mixed, and an appropriate amount of water is added thereto, and the mixture is well kneaded, and is extructed with an extruder into a plate-like sheet, and the resulting sheet is made a spiral-like form thereof with a punching machine to obtain a formulation.

Next, an efficacy of the Type 1 crystal of Compound (1) on controlling harmful arthropods is shown by Test examples. The following tests were conducted at 25°C.

### Test Method 1

Test compound is made to a formulation according to a similar method to that described in the Formulation example 1, and thereto is added water containing 0.03 v/v% of Shindain (registered trademark) to prepare a dilution containing a prescribed concentration of the test compound.

Cucumber (Cucumis sativus) seedling (on the developmental stage of the second true leaf) is planted in a cup, and approximately 30 cotton aphids (*Aphis gossypii*) (all stages of life) are released onto the seedling. After one day, the dilutions are sprayed onto the seedling at a ratio of 10 mL/seedling. After 5 days, the number of the surviving insects is examined, and the controlling value is calculated by the following equation.

Controlling value (%) = {1 - (Cb×Tai)/(Cai×Tb)} × 100 wherein the symbols in the equation represent the following descriptions.
Cb: Number of the test insects in untreated group;
Cai: Number of the surviving insects at the time of the examination in untreated group;
Tb: Number of the test insects in treated group;
Tai: Number of the surviving insects at the time of the examination in treated group;
Here the "untreated group" represents a group where a similar treatment procedure to that of treated group except for not using the test compound is done.

### Test Example 1-1

The test was conducted according to the Test method 1 by making the prescribed concentration 500 ppm and using the Type 1 crystal of Compound (1) as a test compound, and, as the result of the test, the Type 1 crystal of Compound (1) showed 90% or more as the controlling value.

### Test Method 2

Test compound is made to a formulation according to a similar method to that described in the Formulation example 1, and thereto is added water containing 0.03 v/v% of Shindain (registered trademark) to prepare a dilution containing a prescribed concentration of the test compound.

Cabbage (Brassicae oleracea) seedling (on the developmental stage of the second to third true leaf) is planted in a cup, and the dilutions are sprayed onto the seedling at a ratio of 20 mL/seedling. Thereafter, the stem and leaf thereof are cut out and then are placed into a cup that is lined with filter paper. Five (5) commom cutworms (*Spodoptera litura*) at the second instar larval stage are released into the cup. After 5 days, the number of the surviving insects is counted, and the mortality of insects is calculated by the following equation. Mortality (%) = (1 - Number of surviving insects/5) × 100

### Test Example 2-1

The test was conducted according to the Test method 2 by making the prescribed concentration 500 ppm and using the Type 1 crystal of Compound (1) as a test compound, and, as the result of the test, the Type 1 crystal of Compound (1) showed 80% or more as the mortality of insects.

### Test Method 3

Test compound is made to a formulation according to a similar method to that described in the Formulation example 1, and thereto is added water containing 0.03 v/v% of Shindain (registered trademark) to prepare a dilution containing a prescribed concentration of the test compound.

Cabbage (Brassicae oleracea) seedling (on the developmental stage of the second to third true leaf) is planted in a cup, and the dilutions are sprayed onto the seedling at a ratio of 20 mL/seedling. Thereafter, the stem and leaf thereof are cut out and then are placed into a cup that is lined with a filter paper. Five (5) diamondback moths (Plutella xylostella) at the second instar larval stage are released into the cup. After 5 days, the number of the surviving insects is counted, and the mortality of insects is calculated by the following equation. Mortality (%) = (1 - Number of surviving insects/5) × 100

### Test Example 3-1

The test was conducted according to the Test method 3 by making the prescribed concentration 500 ppm and using the Type 1 crystal of Compound (1) as a test compound, and, as the result of the test, the Type 1 crystal of Compound (1) showed 80% or more as the mortality of insects.

### Test Method 4

Test compound is made to a formulation according to a similar method to that described in the Formulation example 1, and thereto is added water to prepare a dilution containing a prescribed concentration of the test compound.

Into the dilution, 30 common house mosquitos (Culex pipiens pallens) at the last instar larval stage are released, and after 1 day, the state of the common house mosquito larvae is examined, and the mortality of insects is calculated by the following equation. Mortality (%) = (Number of dead insects/Number of tested insects) × 100

### Test Example 4-1

The test was conducted according to the Test method 4 by making the prescribed concentration 3.5 ppm and using the Type 1 crystal of Compound (1) as a test compound, and, as the result of the test, the Type 1 crystal of Compound (1) showed 90% or more as the mortality of insects.

### Test Method 5

Test compound is made to a formulation according to a similar method to that described in the Formulation example 1, and thereto is added water containing 0.03 v/v% of Shindain (registered trademark) to prepare a dilution containing a prescribed concentration of the test compound.

Rice (Oryza sativa) seedling (on the developmental stage of the second leaf) is planted in a cup, and the dilutions are sprayed onto the seedling at a ratio of 10 mL/seedling. Thereafter, twenty (20) brown planthoppers (Nilaparvata lugens) at the third instar larval stage are released into the cup. After 6 days, the number of the surviving insects is counted, and the mortality of insects is calculated by the following equation. Mortality (%) = (1 - Number of surviving insects/20) × 100

### Test Example 5-1

The test was conducted according to the Test method 3by making the prescribed concentration 500 ppm and using the Type 1 crystal of Compound (1) as a test compound, and, as the result of the test, the Type 1 crystal of Compound (1) showed 90% or more as the mortality of insects.

### Test Method 6

Test compound is made to a formulation according to a similar method to that described in the Formulation example 1, and thereto is added water containing 0.02 v/v% of Shindain (registered trademark) to prepare a dilution containing a prescribed concentration of the test compound.

Only the second true leaves of Cucumber (Cucumis sativus) seedling (on the developmental stage of the third true leaf) are retained, and the other leaves are cut off, and the dilutions are sprayed onto the seedling at a ratio of 10 mL/seedling. After the dilution, which is treated, is dried, rain is given to the cucumber seedling with an artificial rainfall device at 10 mm/hr for 1 hour. After one day, 10 cotton aphids (Aphis gossypii) female adults are released onto the seedling. After 5 days, the number of the surviving insects is examined and the controlling value is calculated by the following equation.

Controlling value (%) = {1 - (Cb×Tai)/(Cai×Tb)} × 100 wherein the symbols in the equation represent the following descriptions.
Cb: Number of the test insects in untreated group;
Cai: Number of the surviving insects at the time of the examination in untreated group;
Tb: Number of the test insects in treated group;
Tai: Number of the surviving insects at the time of the examination in treated group;
Here the "untreated group" represents a group where a similar treatment procedure to that of treated group except for not using the test compound is done.

### Test Example 6-1

The test was conducted according to the Test method 6 by making the prescribed concentration 1 ppm and using the Type 1 crystal of Compound (1) as a test compound, and as the result of the test, the Type 1 crystal of Compound (1) showed 40.5 % as the controlling value.

### Test Method 7

Test compound is made to a formulation according to a similar method to that described in the Formulation example 1, and thereto is added 1.8 g of sucrose, followed by adding 3% sugar water to prepare a dilution containing a prescribed concentration of the test compound.

A filter paper having a diameter of 5.5 cm is spread on the bottom of the cup, and then 0.7 ml of the dilutons are added dropwise to the filter paper, and ten (10) Argentine ant (Linepithema humile) workers are released into the cup, and the cup is covered with the lid. After 6 days, the number of the dead and distressed individuals of Argentine ant workers are counted, and the ratio of distressed and dead insects (%) is calculated by the following equation.

Here the dead individual is one whose behavior has completely stopped, and a distressed individual is one that cannot stand up when placed on its back and also cannot walk. Ratio of distressed and dead insects (%) = {(Number of dead individuals + Number of distressed individuals)/Number of tested insects} × 100

### Test Example 7-1

The test was conducted according to the Test method 7 by making the prescribed concentration 12.5 ppm and using the Type 1 crystal of Compound (1) and an amorphous of Compound (1) as test compounds, and as the result of the test, the Type 1 crystal of Compound (1) showed 100 % as the ratio of distressed and dead insects. On the other hand, the amorphous of Compound (1) showed 35 % as the ratio of distressed and dead insects.

### Test Method 8

Test compound is made to a formulation according to a similar method to that described in the Formulation example 1, and thereto is added 1.8 g of sucrose, followed by adding 3% sugar water to prepare a dilution containing a prescribed concentration of the test compound.

A filter paper having a diameter of 5.5 cm is spread on the bottom of the cup, and then 0.7 ml of the dilutions are added dropwise to the filter paper, and five (5) German cockroach (Blattella germanica) male adults are released into the cup, and the cup is covered with the lid. After 5 days, the number of the dead and distressed individuals of German cockroach male adults are counted, and the ratio of distressed and dead insects (%) is calculated by the following equation.

Here the dead individual is one whose behavior has completely stopped, and a distressed individual is one that cannot stand up when placed on its back and also cannot walk. Ratio of distressed and dead insects (%) = {(Number of dead individuals + Number of distressed individuals)/Number of tested insects) × 100

### Test Example 8-1

The test was conducted according to the Test method 8 by making the prescribed concentration 0.8 ppm and using the Type 1 crystal of Compound (1) and an amorphous of Compound (1) as test compounds, and as the result of the test, the Type 1 crystal of Compound (1) showed 100 % as the ratio of distressed and dead insects. On the other hand, the amorphous of Compound (1) showed 30 % as the ratio of distressed and dead insects.

### Industrial Applicability

The Type 1 crystal of Compound (1) as the compound of the present invention shows excellent control efficacy against harmful organism.

## Claims

1. A Type 1 crystal of 2-(5-cyclopropyl-3-(ethylsulfonyl)pyridin-2-yl)-5-((trifluoromethyl)sulfonyl)benzo[d]oxazole, wherein the crystal has diffraction peaks at 2θ = 14.3±0.2°, 15.6±0.2°, 17.1±0.2°, 18.7±0.2°, 20.1±0.2°, 22.4±0.2°, 23.3±0.2°, 24.8±0.2°, 26.1±0.2°, and 28.5±0.2° in a powder X-ray diffraction with Cu-Kα radiation.

2. The Type 1 crystal according to claim 1 wherein a lattice constant measured by single crystal X-ray diffraction is indicated below:
a = 6.45Å±0.01 Å, b = 14.55 Å±0.01 Å,
c = 20.86 Å±0.01 Å, α = β =γ = 90°.

3. The Type 1 crystal according to claim 1 or 2, wherein the crystal has peaks at positions of 1370±4cm⁻¹, 1317±4cm⁻¹, 1190±4cm⁻¹, 1124±4cm⁻¹, 1093±4cm⁻¹, 1047±4cm⁻¹, 1034±4cm⁻¹, 846±4cm⁻¹, 826±4cm⁻¹, 776±4cm⁻¹, 738±4cm⁻¹ and 719±4cm⁻¹ as a wavenumber in an infrared absorption spectrum (ATR method).

4. The Type 1 crystal according to any one of claims 1 to 3, wherein a melting point is 180 to 182 °C.

5. A method for preparing the Type 1 crystal according to any one of claims 1 to 4, which comprises
a step of dissolving 2-(5-cyclopropyl-3-(ethylsulfonyl)pyridin-2-yl)-5-((trifluoromethyl)sulfonyl)benzo[d]oxazole that melts at a range of 175 to 178°C into one or more solvents selected from the group consisting of hydrocarbon, alcohol, ether and ketone, and
a step of cooling the resulting solution to precipitate a crystal.

6. A harmful organism control composition comprising the Type 1 crystal according to any one of claims 1 to 4.

7. A composition which comprises one or more ingredients selected from the group consisting of the following Groups (a), (b), (c) and (d), as well as the Type 1 crystal according to any one of claims 1 to 4:
Group (a): a group consisting of insecticidal ingredients, miticidal ingredients, and nematicidal ingredients;
Group (b): fungicidal ingredients;
Group (c): plant growth modulating ingredients; and
Group (d): repellent ingredients.

8. A method for controlling a harmful organism which comprises applying an effective amount of the Type 1 crystal according to any one of claims 1 to 4 or an effective amount of the harmful organism control composition according to claim 6 to a harmful organism or a habitat where the harmful organism lives.

9. A seed or vegetative reproductive organ carrying an effective amount of the Type 1 crystal according to any one of claims 1 to 4 or an effective amount of the composition according to claim 6.

10. Use of the Type 1 crystal according to any one of claims 1 to 4 for controlling a harmful organism.
